# EUROPEAN PATENT APPLICATION

(11) **EP 4 268 582 A1**
(43) Date of publication of application: **01.11.2023**
(21) Application number: 22169951.5
(22) Date of filing: 26.04.2022
(51) Int. Cl.: A01G 33/00, A01H 4/00, C12M 1/00, C12N 1/12, C12P 19/04

(54) **METHODS AND DEVICES FOR CULTIVATING COENOCYTIC ALGAE**

(71) Applicant: Ecole Polytechnique Fédérale de Lausanne (EPFL) EPFL-TTO, 1015 Lausanne (CH)
(72) Inventor: NICODEME, Eya, 1025 St-Sulpice VD (CH); ZANCHETTA, Enio, 1022 Chavannes-Renens (CH); LUDWIG, Christian, 5400 Baden (CH)
(74) Representative: Schneiter, Sorin

(57) **Abstract**

The present invention concerns methods and devices for cultivating coenocytic algae and for producing cellulose, in particular microfibrillated cellulose (MFC). In one embodiment, the algae are grown on a solid substrate immersed in liquid culture medium. The solid substrate preferably comprises a carbonite mineral. The invention also concerns a method for producing spores of said coenocytic algae and a device comprising two separate containers, a first container for growing said algae and a second container for growing larger algae and inducing the generation of spores.

## Description

### Technical Field

The present invention relates to methods and devices for cultivating algae and in particular coenocytic algae. The invention further relates to the production of cellulose.

### Background art and problems underlying the invention

For producing products that meet human's needs, raw materials are obtained and subjected to the appropriate transformation processes. Raw materials, such as petroleum, may be obtained by extraction from the Earth's crust or soil. Other raw materials, such as cellulose, may be obtained from the biosphere, for example from wild growing woods or from cultivated crops. In both cases, the obtention of raw materials is associated with important disadvantages, that are the subject of fundamental challenges humanity as a whole is currently faced with. Extraction of petroleum for producing consumer products, such as packaging for all sorts of goods, is ultimately associated with the release of carbon dioxide into the atmosphere, and the contribution to global warming. Using trees for extracting raw materials, such as cellulose, also leads to release of CO₂. Finally, cultivation of crops for obtaining certain raw materials that are other than food is also not an ideal solution, due do the limited terrestrial surfaces that are suitable for crop growing, the role of arboreous surfaces in maintaining the climate within a habitable range and contributing to reliable rainfall, and the growing size of the human population requiring food.

Biotechnological processes, which take place under controlled conditions and generally in a more or less closed system, are generally used for in the production specific, important molecules having valuable biological activity of for producing various types of fermented food. Biotechnological processes are rarely used for producing raw materials, probably because such processes are currently not suitable to produce large amounts required for many raw materials.

One objective of the present invention is to provide a method for obtaining raw materials that can be used for packaging. Packaging is frequently made from plastic materials, which entails the problems depicted above. Another raw material suitable for packaging and other products is cellulose, in particular nanocellulose. Cellulose has a wide range of applications and might involve many industrial fields such as composites and plastics, medical and pharmaceutics, paper and paperboard, paints and coatings, food science, packaging, cement, textile, electronics, filtration, adhesives, aerogels, sensors, batteries, cosmetics or biofuels, just to mention a few.

Cellulose is, with hemicellulose and lignin, a component present in plant cells and is a key structural component of the cell walls of these organisms. Microfibrillated cellulose (MFC, a new form of engineered biomass) and bioplastics containing cellulose are intensively studied as potential replacements for some fossil-based plastics due to their recyclability and biodegradability. However, even if sourcing cellulose materials from forests and crops appear attractive due to the renewable nature of this resource, there are hiding questions regarding the reliance on forests for future material needs. This can also present a threat to the world's food supply as fertile lands are generally needed to grow trees and crops. Moreover, MFC production from wood is an energy-intensive process with a moderated yield of extraction due to the presence of lignin and hemiacetal. Therefore, the MFC industry must deal with the vitally important question of how to produce feedstock.

In view of the above, it is an objective of the invention to provide a method for producing raw materials and other products of human need in a manner that is renewable and allows to address the problems depicted above.

On objective of the invention is to provide a method for producing cellulose. Certain algae produce cellulose as part of their cell wall. Photosynthetic multinucleate cells are unique algae containing a thick cell wall composed of microfibrils of cellulose arranged in a complex structure of parallel and crossing fibres. Some coenocytic algae, such as *Valonia sp,* have very crystalline cellulose in the cell wall. The state of the art of the coenocytic algae, their growth regime, cultivation methods are limited, as there is no interest in coenocytic algae industrialization so far.

It is an objective of the invention to determine whether it is possible to cultivate algae for producing raw materials, such as cellulose, or bioactive compounds. It is an objective to determine whether algae can be used and/or cultivated to produce sufficiently large quantities, and whether such raw materials have any suitable use, for example in the production of packaging.

It is an objective of the invention to find a way for cultivating coenocytic algae for the purpose of obtaining a material that is of use for human needs.

### Summary of the Invention

Remarkably, the present inventors have provided devices and methods for cultivating algae, in particular coenocytic algae.

The meaning of coenocyte (koinós = common; kýtos= box) is, as its Greek etymology indicates, a multinucleate cell. Coenocyte cells are formed when a cell performs nuclear division without cytokinesis. Most coenocytic algae are part of the *Chlorophyta* and *Caulerpales* taxonomic groups. Because of their unique properties, they are visible and can, in some cases, reach a few centimetres. Their most common habitats are the tropical and subtropical waters, not living in cold waters. Their depth range is not something specific, and they can live from mid-intertidal level up to a hundred meters deep in the sea.

Coenocytic algae produce microfibrillated cellulose (MFC). MFC is available in every biomass producing cellulose: trees, cotton, bacteria, algae and even some animals. Plants produce this material with different a wide range of purity. For instance, corn's composition is made of 35% of cellulose, while cotton reaches higher values like 95% purity. Plant MFC has high availability and can be directly harvested from the wild. However, because the cellulose content varies significantly between the plants, the methods must be specific and adapted for the plants used, complexifying the process. Algae microfibrillated cellulose has higher crystallinity than plant-based MFC due to both its high degree of polymerization (26500 DP) and its thick microfibrils (10-30 nm) (N. Peneder, What is microfibrillated cellulose (MFC)? - Weidmann Fiber Technology, Weidman. (2017)). Gels produced from algae fibrillated cellulose are suitable for packaging because of their strong rheological properties, higher specific surface area and robustness (A. Mihranyan, K. Edsman, M. Strømme, Rheological properties of cellulose hydrogels prepared from Cladophora cellulose powder, Food Hydrocoll. 21 (2007) 267-272).

Many algae have a high cellulose content and purity; the absence of lignin removes several steps in the MFC manufacturing compared to wooden biomass or perennial plants.

Some unique algae species, such as coenocytic algae, contain a high amount of long and pure microfibrillated cellulose. MFC industries rely mainly on wood and cotton as they exhibit 40 to 50% and up to 90% cellulose content, respectively. Even if these sources are abundant, intensive use of them can drive some countries to deforestation.

**Table 1: Dimensions of cellulose nanocrystals**

| **Biomass** | **Length [nm]** | **Width [nm]** | **Aspect ratio (LID)** |
|---|---|---|---|
| Wood | 100-300 | 3-5 | 20-100 |
| Cotton | 100-150 | 5-10 | 10-30 |
| Ramie | 70-200 | 5-15 | N/A-12 |
| Valonia (Coenocyte algae) | 1000-2000 | 10-20 | 50-200 |
| Tunicates | >1000 | 10-20 | N/A -100 |
| Bacteria | 160-420 | 10-50 | 2-100 |

Industrial production of algae for MFC extraction can offer new perspectives to the bioplastic industries. This is possible as algae do not require fertile land, and marine water can be used as the main source for nutrient and water supply. Moreover, cellulose content in the cell wall of coenocytic algae can reach up to 70% DW.

In an aspect, the invention provides a method for cultivating, growing and/or producing algae, in particular coenocytic algae, the method comprising: providing a solid substrate comprising coenocytic algae, and, cultivating said algae by exposing said substrate to a liquid culture medium.

In an aspect, the invention provides a method for cultivating growing and/or producing algae, in particular coenocytic algae, by way of a solid-liquid culture.

In an aspect, the invention provides a device for cultivating, growing and/or producing algae, in particular coenocytic algae, the device comprising: a container suitable to retain a liquid culture medium; one or more solid substrates suitable to be placed in said container, wherein said solid substrate is suitable for growing said algae thereon; channels and/or tubes suitable for feeding gaseous CO₂ and/or air into the container and more preferably to said liquid culture medium in said container; a heater for heating said liquid culture medium, and, optionally: one or more light sources for illuminating algae growing on said solid substrate.

In an aspect, the invention provides a method for producing algae, in particular coenocytic algae, containing spores and/or for producing spores of such algae, the method comprising:
providing cells of said algae and separating said cells in at least two groups on the basis of the size of said cells, a first group comprising comparatively larger cells and a second group comprising comparatively smaller cells;
cultivating said smaller cells in a liquid medium in first container;
exposing said larger cells to mechanical stress and continuing cultivating said larger cells in a second container;
removing cells comprising spores from said second container and separating said spores from said removed cells.

In an aspect, the invention provides a device for producing algae containing spores and/or for producing spores of such algae, in particular coenocytic algae, the device comprising: first and second containers suitable for cultivating said algae in a liquid culture medium; a transfer device, suitable for transferring cells from said first to said second container; a separating device, suitable for separating larger cells from smaller cells; and, a cell removing device, suitable to remove spore-containing cells from said second container. Preferably, said cell removing device is an automated cell removal device.

In an aspect, the invention provides a method comprising both methods sequentially: the method for producing algae comprising spores and the method for cultivating algae, wherein said algae are preferably cultivated from said spores, for example after inoculating said spores on a substrate.

In an aspect, the invention provides a system comprising on or both of the devices of the invention and a data processing entity. The data processing entity is preferably configured to conduct one or more steps of the methods of the invention, preferably in an automated or semi-automated, computer-assisted manner.

In an aspect, the invention provides the use of a solid substrate for cultivating algae, in particular coenocytic algae.

In an aspect, the invention provides a method for producing cellulose, the method comprising: cultivating algae, in particular coenocytic algae, preferably according to the present invention; harvesting said cultivated coenocytic algae; and, extracting cellulose from said harvested algae.

Further aspects and preferred embodiments of the invention are defined herein below and in the appended claims. Further features and advantages of the invention will become apparent to the skilled person from the description of the preferred embodiments given below.

### Brief Description of the Drawings

**Figure 1** shows photographs of *Boergesnia forbesi* grown at 28°C in presence of acetate as a carbon source. The left photograph shows a cell at the beginning and the right photograph at the same cell at the end of the experiment.
**Figure 2** is a graph showing the pH at the end of growth experiments conducted with *Boergesenia forbesi* as described in the experimental section.
**Figure 3** shows photographs of solid-liquid cultures of *Valonia,* with (left Erlenmeyer flask on photo) and without CaCO₃ substrate, at the beginning (left photo) and at the end of the experiment (right photo).
**Figures 4A-4E** show a device for producing spores and illustrate a method for producing coenocytic algae containing spores in accordance with embodiments of the invention.
**Figure 5** illustrate an embodiment of the substrate of the invention.
**Figure 6** shows a device for cultivating coenocytic algae in solid-liquid culture in accordance with an embodiment of the invention.

Hereinafter, preferred embodiments of the device of the invention are described, in order to illustrate the invention, without any intention to limit the scope of the present invention.

### Detailed Description of the Preferred Embodiments

The present invention relates to methods and devices for cultivating algae. Preferably, said algae are coenocytic algae and/or green algae.

Preferably, said coenocytic algae are selected from coenocytic green algae, preferably from the phylum of chlorophyta, which comprises the subgroups (orders) of the *Chlorococales,* the *Cladophorales* and the *Caulerpales,* In a preferred embodiment, said coenocytic algae are selected from the group (order) of the *Cladophorales* and *Siphonocladales,* which preferably comprise the families of the *Cladophoaceae,* the *Sipohonocladaceae,* and the *Valoniaceae.*

In a preferred embodiment, said coenocytic algae are selected from *Cladophora, Chaetomorpha, Rhizoclonium, Microdyction, Valonia, Dictyosphaeria, Siphonocladus,* and *Boergesenia,* more preferably from *Valonia, Boergesenia, Rhizoclonium, Chaetomorpha,* and *Cladospora,* for example from *Valonia,* and *Boergesenia.*

In a most preferred embodiment, said algae are selected from *Boergesenia sp.* and *Valonia sp,* for example from *Valonia ventricosa* and *Boergesenia forbesi.*

The method for cultivating said algae is preferably suitable for up-scaling to an industrial scale, enabling the mass production of said algae and/or of raw materials or components, such as bioactive molecules, comprised in said algae.

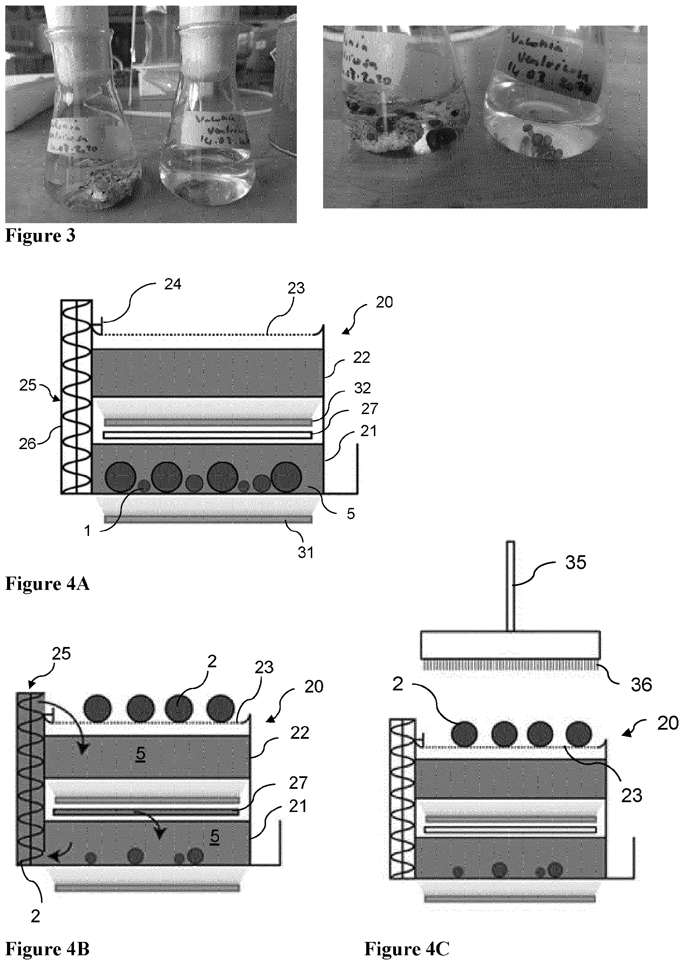

The invention is also directed to a method and a device for obtaining algae comprising spores and/or for producing said spores. In an embodiment, said spores are aplanospores. The method and device for obtaining spores may also be referred to as the preculture or pre-cultivating of said algae, as the obtention of spores may be considered as advantageous for cultivating the algae on a large scale. The device and method for producing said spores will be discussed herein after with reference to Figures 4A to 4E.

**Figure 4A** shows a device or system 20 for producing algae comprising spores. The device comprises two recipients or containers, 21, 22. In the embodiment shown, a first container 21 is provided on the bottom and a second container 22 is provided vertically above said first container. Of course, other dispositions of the containers, including side-by-side and/or partially overlapping arrangements, are encompassed by the invention. The containers are suitable for receiving and/or retaining a liquid, preferably aqueous culture medium 5. The device 20 preferably comprises equipment necessary for maintaining conditions that are conducive to the growth of the algae. Such equipment (not shown in the figure) preferably encompasses tubes, pipes, ducts and/or channels for delivering fresh medium and/or other nutrients, air (Oz) and preferably carbon dioxide (CO₂), for removing used liquid medium and/or for moving the medium, such as stirring or the creation of flow streams within the container. Such equipment preferably also comprises tubing and/or channelling for removing used medium and/or for filtering medium, and the like.

In an embodiment, the device 20 comprises one or more light sources 31, 32 for illuminating cells in said first and second container and for supporting growth of said algae in said containers. In the embodiment shown, two light sources 31, 32 are shown, one for each of the two containers. In the embodiment shown, the containers 21, 22 are transparent or comprise a bottom surface comprising a transparent material, and the light sources 31, 32 illuminate the respective containers 21, 22 from the bottom.

It is noted that the invention also encompasses using sunlight for providing light for supporting the growth of the algae. In this case, one or more separate light sources may be absent. This may be the case with outdoor methods and/or devices, and/or methods conducted in a greenhouse, for example.

It is noted at this stage that the invention encompasses open and closed cultivations systems, both, for producing spores and/or for cultivating algae in order to obtain biomass.

In a preferred embodiment, the device 10 comprises a transfer device 25, suitable for transferring cells from said first container 21 to said second container 22 and vice versa. The transfer device may comprise one or more transfer channels or pipes 26, 27, suitable to transfer medium containing cells of said algae from one container to another container, for example.

In an embodiment, the device 20 comprises first and second transfer channels 26, 27, said first transfer channel being provided to transfer cells from said first container to said second container, preferably by transferring liquid medium containing said cells from said first to said second container; and, said second cell transfer channels 27 being provided to transfer cells, preferably by transferring liquid medium containing said cells, from said second container to said first container. As will be discussed below, a separation step is preferably conducted, such that only smaller cells 1 will be transported back via said second transfer channel 27.

In an embodiment, said first and second cell transfer channels comprise separate pipes, paths and/or channels for liquid transport.

In an embodiment, one selected from said first and second cell transfer channels uses gravity as driving force for transferring cells and/or medium between the containers.

In the embodiment shown, the first transfer channel 26 is equipped with a motorized mechanism, such as a pump, suitable for actively transporting the medium, for example against the force of gravity and/or for overcoming inertia. As the second container is situated vertically above the first container, the second transfer channel 27 exploits gravity for transferring medium containing cells from said second to said first container. The invention encompasses an inverse arrangement (first container above the second), and/or both transfer channels being equipped with pumps for active transport of medium and/or algae cells contained therein.

In an embodiment, the device 20 comprises a separating device 23, suitable for separating cells based on a size of the cells. Preferably, the separating device 23 is suitable for separating comparatively larger cells 2 (Fig. 4B) from comparatively smaller cells 1.

In an embodiment, said separating device 23 comprises a sieve, suitable for separating cells on the basis of their size.

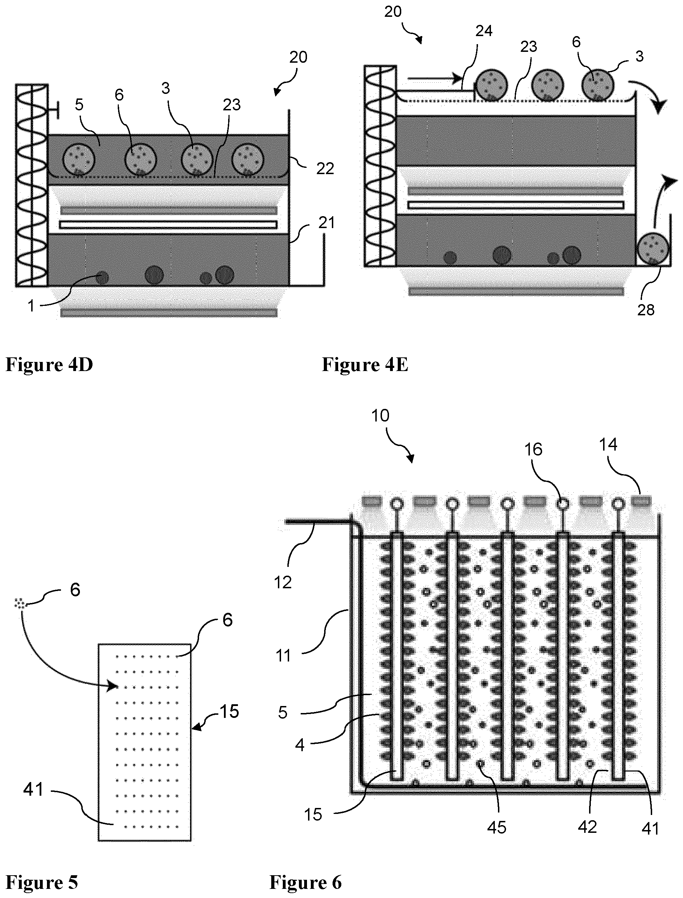

Preferably, said separating device is movable and/or configured to move, preferably in an automated manner. In the embodiment shown, the separating device 23 is configured to move from a bottom of the second container (Fig. 4D) to the top and/or out of the container (Figs 4A-4C, 4E), so as to remove cells out of the medium 5 contained in the second container. Preferably, the device comprises a motor (not shown) for driving the separating device 23.

As visible in Fig. 4C, the device 20 comprises, according to a preferred embodiment, a stress inducing entity 35 for exposing said cells to stress. In a preferred embodiment, the stress inducing entity 35 is suitable to induce mechanical stress on algae cells.

In an embodiment, the stress inducing entity preferably comprising a plurality of spikes 36. Preferably, the stress inducing entity is motorized and is provided movable under the control of said motor. The motor for driving the stress inducing device is not shown in the figures.

As best seen in Fig. 4E, the device 20 comprises, according to a preferred embodiment, a cell removing device 24. The cell removing device is preferably suitable to remove cells from said second container, in particular spore-containing cells. In the embodiment shown, the cell removing device 24 works in combination with the separating device 23 for removing the cells from the medium and/or transferring the cells, for example, to a transfer device 28, from which the cells containing spores can be transported, preferably in an automated manner, for further use.

In an embodiment, the cell removing device 24 comprises a wiper and/or a scraper, provided to wipe and/or scrape cells from said second container, preferably from the sieve. Preferably, the cell removing device 24 is motorized.

Preferably, the device 20 and/or several or all of its components are operating in an automated manner, for example in a computer-implemented process. The invention also encompasses a data processing entity (not shown) for operating the device 20. Preferably, the device and the data processing entity provide a system for producing algae comprising spores.

Preferably, one, several or all of the components, such one or more pumps of the transfer device 25, the separating device 23, the cell removing device 24, and, in as far as present, artificial light sources 31, 32, are under the control of the data processing unit. Preferably, one or more pumps and/or moving components, such as the transfer device 25 and/or the separating device 23, are motorized and preferably under the control of one or more electric motors, which motors are under the control of said data processing entity.

For example, the step of filtering the algae by way of the separating device 23 could be conducted autonomously, for example once the system determines that a certain quantity of large algae are present, or simply at regular intervals.

For example, the system could activate automatically one or more pumps to transfer medium and algae cells contained therein through the first transfer channel 26 and through the sieve 23.

Other steps, such as one or more steps selected from: activating the stress inducing device 35, lifting the sieve 23, and activating the cell removing device 24, may also be conducted in an automated or semi-automatic manner, preferably under control of and/or assisted by the data processing entity.

Having discussed the exemplary device for obtaining cells of algae containing spores, in particular cells of coenocytic algae, the method for producing spores of said algae can be explained with reference to Figs 4A-4E.

In an embodiment, the method comprises providing cells of said algae and separating said cells in at least two groups on the basis of the size of said cells, a first group comprising comparatively larger cells 2 and a second group comprising comparatively smaller cells 1.

In a preferred embodiment, cells 1 of the algae are grown in the first container 21 before separating said cells in two groups. As can be seen in Fig. 4A, cells 1 are cultivated in medium 5 in a first container 21.

Once the cells comprise cells 2 having the required size, said cells are separated from the remaining cells 1, which are not yet suitable for spore production.

In order to conduct the separation, the medium comprising the cells is transported, as illustrated in Fig. 4B, from the first container to the second container via the first transport channel 26 of the transfer device.

In a preferred embodiment, separating said cells comprises filtering said larger cells 2.

The second container 22 is equipped with a cell separating device 23, in particular a sieve. Cells 2 having the required size are retained in the sieve 23, as shown in Fig. 4B.

In an embodiment, cells having a size (diameter) of 0.5cm or larger, preferably 1cm or larger, more preferably 1.5cm or larger, and most preferably 1.8cm or larger are separated by said cell separating device, preferably said sieve. These sizes can be determined by selecting the appropriate mesh size of the sieve, and can thus be equalled with said mesh size. In other words, cells my be separated with a sieve having mesh size of 0.5 or larger, etc.

Smaller cells 1 pass through the sieve with the medium and to the second container 22, from where they are transported, with the medium, via the second cell transfer channels 27 back to the first container 21. The entire process of medium and/or cell transport and separation is illustrated in Fig. 4B with the aid of the arrows, indicating the direction of the flow of the medium 5.

Being back in the first container, growth of said smaller cells is continued by cultivating said cells 1 in said first container.

In a preferred embodiment, the method of the invention comprises exposing said larger cells 2 (or said cells meeting the appropriate size requirement) to mechanical stress and continuing cultivating said larger cells in a second container 22.

Accordingly, the cells 2 having the appropriate size, selected by the separation step, are exposed to a stress treatment as shown in Fig. 4C. In the embodiment shown, a mechanical stress device 35 is used. The device comprises a plurality of spikes 36. The spikes are preferably provided substantially in parallel and/or next to each other, preferably providing an array of spikes. In the embodiment shown, the cells 2 are outside the medium and the container and are exposed on the sieve 23, which is lifted outside the container 22. The mechanical stress inducing entity 35 is lowered towards the cells on the sieve, such that the spikes 36 get in contact with the cells and preferably pierce, squeeze and/or slightly damage the cells. Thereafter, the stress inducing entity 35 is lifted so as to leave the cells on the sieve 23, which is lowered into the second container, as shown in Fig. 4D.

In an embodiment, the method comprises cultivating said cells 3 exposed to stress in said second container. As shown in Fig. 4D, cells are cultivated simultaneously in said first and second containers 21, 22: The first container may be used to cultivate cells 1 until they have a determined seize suitable for the induction of spore generation, and the second container 22 may be used for cultivating cells exposed to stress for allowing the generation of spores 6 in said stress-induced cells 3 of said algae.

The exposure of the cells 2 to stress induces the formation of spores in these cells. The cells in the second container are thus continued to be cultivated in an appropriate medium 5, until the formation of spores has sufficiently advanced for allowing extraction of spores.

In a preferred embodiment, the method of the invention comprises removing cells 3 comprising spores 6.

To this end, the device 20 comprises the cell removing device 24. The device 24 is suitable for assisting in the removal of spore-containing cells 3 from said second container and/or for transferring cells from the separating device 23.

The cell removing device 24 is preferably a wiper and/or a scraper.

In the embodiment shown, the cell separating device 23 is lifted out of the container, and the cell removing device 24, which is preferably a wiper, scrapes off the algae 3 from the cell separating device, which is preferably said sieve. In the embodiment shown, the wiper pushes the cells 3 out of the cell separating device 23 and into a transfer device, such as a channel 28, which may comprise a conveyor belt, for transporting said cells 3 for further use.

In Figures 4D and 4E, the reference numeral 3 is used to show the cells having spores 6 formed inside the cells. Once they are removed from the device, for example as described, the spores can be separated from the cells. This can be conducted by rupturing the cells, if necessary, and by centrifugation. Centrifugation is suitable separates the spores and cell debris.

Preferably, cell debris is transferred for further use, for example cellulose extraction, for example es disclosed elsewhere in this specification.

The spores 6 may be used for inoculating a substrate 15 in accordance with an embodiment of the invention, as shown in **Figure 5****.**

The substrate 15 may be used for cultivating the algae, preferably in a solid-liquid culture. In other words, algae 4 are growing on the substrate 15, when the substrate is immersed in a liquid culture medium 5, as illustrated in **Figure 6****.**

In an embodiment, the solid substrate 15 comprises a carbonate mineral. Preferably, the algae grow on said carbonate mineral.

In an embodiment, the solid substrate 15 comprises calcium carbonate (CaCO₃).

In an embodiment, the solid substrate comprises one selected from the group consisting of calcite, aragonite and vaterite, preferably at the surface of the substrate.

In an embodiment, the solid substrate comprises said carbonate mineral deposited on a solid carrier material. The solid carrier material may comprise or essentially consist of any suitable material, in particular any material that allows for the deposition of said carbonate mineral and the immersion into water for the purpose of said solid-liquid culture. In an embodiment, the substrate comprises a material selected from minerals, metals (e.g. aluminium or stainless steel), an artificial material, such as plastic, alloys, ceramics and composites. In an embodiment, the solid carrier material comprises SiO₂. In a preferred embodiment, the solid carrier material comprises and/or consists essentially of glass.

In an embodiment, the solid carrier material comprises negatively charged and/or hydroxylated substrates. The hydroxylated or negatively charged substrate facilitates the deposition of a carbonate mineral. In a preferred embodiment, the solid carrier material comprises negatively charged and/or hydroxylated glass.

In an embodiment, the solid substrate comprises glass coated with said carbonate mineral. Preferably, said solid substrate comprises glass comprising a hydroxylated and/or negatively charged surface, with said carbonate mineral being coated on said hydroxylated and/or negatively charged surface.

In an embodiment, the solid substrate comprises a plate comprising two, first and second, opposed main surfaces 41, 42, for example front and back surfaces. Said opposed surfaces preferably have about the same surface size. Preferably, algae are grown on both said first and second sides. Preferably, said main surfaces provide the major part of the surfaces of said substrate, preferably at least 70% of the surface of said substrate.

Preferably, said substrate is inoculated with said spores 6, which are deposited on a surface of the substrate, preferably on one or both of the two opposed surfaces.

In an embodiment, the solid substrate comprises or consists essentially of an outline selected from polygonal and curved outlines, or an outline comprising both, straight and curved sides. The substrate may comprise a circular, elliptical or otherwise curved outline. In a preferred embodiment, the solid substrate comprises a polygonal outline selected from quadrilateral, pentagonal and hexagonal forms. Most preferably, the solid substrate comprises a rectangular outline.

The form and/or outline of the solid substrate is preferably selected so as to enable the use of a plurality of substrate in a cultivating tank 11, as shown in Fig. 6. In particular, the outline and thickness are preferably selected so as to optimize the surface that is available for culture, preferably solid-liquid culture.

In an embodiment, the first and second opposed surfaces 41, 42 of said solid substrate are preferably flat surfaces.

In a preferred embodiment, said substrate is porous and/or comprises one or more porous surfaces, preferably microporous surfaces. Preferably, said mineral surface, in particular said carbonate mineral surface, is provided so as to form said porous, preferably microporous surface. The porosity of the surface is preferably selected so as to facilitate the self-adhesion and/or rooting of said algae on the substrate. Nano- or microporous surfaces may be suitable.

In an embodiment, the surface comprises pores having an average size of 0.1-1'000 µm, preferably 0.5-500 µm, even more preferably 1-200 µm, and most preferably 1-50 µm. In an embodiment, the porosity is 5-50 µm. In yet another embodiment the porosity is 10-20 µm. Porosity may be determined with any method, or combination of methods, that is suitable to measure pores sizes at the indicated range. Pore size measurement with AFM (atomic force microscopy) and SAXS (small-angle X-ray scattering) is preferred, for example as disclosed in Fantazzi et al, "Gains and losses of coral skeletal porosity changes with ocean acidification acclimation", Nature Communications, 6:7785; DOI: 10.1038/ncomms8785.

The present invention comprises methods and devices for cultivating, growing and/or producing algae, in particular coenocytic algae. Preferably, said methods favour vegetative growth of said algae, as opposed to other methods disclosed herein, which are more adapted to the production of spores. In a preferred embodiment, cultivating said algae comprises producing biomass of said algae and/or increasing the biomass of said algae.

Figure 6 discloses an exemplary device 10 for cultivating algae, in particular by solid-liquid culture. The device 10 comprises a container, tank or other suitable reservoir 11, which is suitable to receive and/or retain a liquid, preferably aqueous medium 5, and to receive the solid substrates 15 of which preferred embodiments have been described elsewhere in this specification.

In the embodiment shown, a plurality of substrates 15 are immersed in the container 11. Algae 4 are shown to grow vegetatively on the two opposed surfaces 41, 42 of the substrates.

In a preferred embodiment, the device 10 comprises a mechanical arrangement 16 suitable to maintain one or more solid substrates 15 in the container.

As shown in Fig. 10, the device preferably comprises one or more artificial light sources 14, suitable to illuminate said algae so as to provide conditions that optimize growth and/or photosynthetic assimilation. In some embodiments, sunlight may be used for illuminating the algae 4, in which case artificial light sources 14 may be absent.

In an embodiment, the device comprises one or more heating element (not shown), for heating the medium 5 in the container to the optimum value, for example, again with the goal of providing optimum growth conditions. One or more heating elements may also be present in the device for producing algae comprising spores, for example in the device shown in Figs 4A-4E.

The device 10 is preferably further equipped with conducts, lines, pipes and/or tubing 12 or delivering nutrients, oxygen and/or CO₂ into the container. Oxygen may be provided by adding air through the appropriate tubing, for example. In Fig. 6, the tubing 12 illustrates the delivery of air and/or CO₂ 45 into the container.

One or more pipes, tubes and/or conducts may also be provided, preferably in combination with one or more pumps, for transporting, stirring and/or moving said liquid medium 5. Movement of the medium within the container 11 is advantageous so as to favour diffusion of nutrients within the container. Furthermore, the medium may be transported out and/or into the container of the container for filtering, cleaning and/or exchange with new medium.

In an embodiment, the method of cultivating algae comprises providing a solid substrate 15 comprising algae, in particular coenocytic algae.

In an embodiment, obtaining said solid substrate 15 comprising algae comprises inoculating a solid substrate with spores 6 of said algae. The inoculation of the solid substrates 15 with spores 6 of said algae is illustrated in Fig. 5.

In an embodiment, the liquid culture medium is supplemented with CO₂ and/or air during cultivation in said liquid culture medium, for example as described above with reference to Fig. 6.

In an embodiment, the liquid medium comprises nutrients suitable for supporting growth of said algae. Such nutrients preferably include one or more selected from the group of vitamins, minerals, a source of nitrogen, a source of phosphorous and a source of carbon. An exemplary source of nitrogen is a source of NO₃⁻. An exemplary medium suitable for favouring growth in the methods of the invention is disclosed in the Examples herein below.

In an embodiment, the medium is supplemented with a source of carbon. An exemplary source of carbon is acetate. In some embodiments, carbon is added in the form of CO₂, which may be bubbled into the container 11 via appropriate tubing.

In a preferred embodiment, the medium is an aqueous medium. The medium may comprise one selected from sweet and saline water.

Preferably, the medium comprises saline water, in particular water comprising dissolved NaCl. Preferably, the medium has a salinity of 0.1% or larger (1 g salt per kg of water), more preferably 0.5%, 0.6%, 0.7%, 0.8%, or 0.9% or larger.

In an embodiment, the medium has a salinity of 2% or larger, preferably 2.5% or larger, more preferably 3.0% or larger, for example about 3.5%.

In a preferred embodiment, the liquid culture medium comprises sea water. Sea water has generally a salinity of 1.0% or larger. The average salt content of oceans is 3.5% (35 g salt per kg of water).

In an embodiment, the invention provides the use of sea water for cultivating algae, in particular coenocytic algae.

In a preferred embodiment, when cultivating said algae, the temperature of the medium is at a temperature of 20°C or higher, preferably 23-35°C, more preferably 27-30°C. In a preferred embodiment, the temperature is kept at 25-30°C, for at least part of the time, preferably most of the time. Preferably, the temperature is adjusted and/or controlled by suitable equipment, which may comprise heating elements and/or cooling elements.

In a preferred embodiment, cultivating said algae comprises growing said algae at a growth rate that is 2% or more of daily increase of biomass, preferably 3% or more, more preferably 4% or more, and most preferably 5% or more of daily biomass increase. In a most preferred embodiment, the algae are grown at a growth rate of 6% or more of daily increase of biomass.

As used herein, the term "biomass" refers to material produced by growth and/or propagation of cells. Biomass may contain cells and/or intracellular contents as well as extracellular material. Extracellular material includes, but is not limited to, compounds (i.e., cellulose, microfibrillated cellulose) secreted by algae cells. Preferably, water is removed for measuring the biomass, such that biomass is substantially determined on the basis of dry matter.

As has been described with respect to the method for producing spores, the method and/or device for cultivating the algae may be computer-assisted and/or may be conducted in a semi-automatic or automated way. In particular, the device 10 may be part of a system comprising the device and a data processing entity configured to automatically conduct one or more steps of the method of the invention, and/or other steps not disclosed herein but assisting the operation of the device. For example, parameters of the medium, such as salinity, contents of nutrients, pH, medium flow speed and type, oxygen and/or CO₂ content, and the like, may be controlled in an automated manner. One or more selected from removal of the solid substrate from the container and/or harvesting of algae may also be conducted in a semi-automated and/or automated manner. Motors and/or pumps which may be present for assisting in the operation of the system and/or conducting the method of the invention are not shown.

In some aspects, the invention concerns a method for producing cellulose. The cellulose is preferably extracted from the algae that are produced in accordance with the invention, for example as disclosed throughout this specification.

The cultivated algae are preferably harvested, for example by removal from the solid substrates 15, once the algae have attained a target indicator, such as a target size, target biomass, and the like.

In an embodiment, harvesting said cultivated algae comprises, in particular coenocytic algae, mechanically removing biomass of said algae from said solid substrate.

Preferably, with reference to Fig. 6, the solid substrate 15 is removed from the container 11, for example lifted out of the container, for example in a manual or automated manner, so as to facilitate the removal of the algae.

In some embodiments, the device 10 comprises motors, preferably electric motors (not shown) as appropriate for removing the one or more substrates 15 from the container.

In an embodiment, mechanically removing biomass comprises removing algae cells from said substrate while leaving roots of the algae in or on the substrate.

When harvesting said algae from said solid substrates, parts of the algae, in particular the roots, are preferably kept on the substrate.

For example, a cutting tool, such as a knife or a mower, may be used for removing biomass of said algae from the substrate.

In an embodiment, the method comprises extracting cellulose from said harvested algae. Various protocols are available for extracting cellulose from algae, such as coenocytic algae.

According to an embodiment, algae cells are crushed and cell debris is further processed for cellulose extraction. For example, cell walls are boiled, preferably in distilled water, and then in 0.1 N NaOH, preferably under inert ambiance, such as under nitrogen. The purified cell walls may then be rinsed with distilled water and dried, thereby yielding the cellulose.

Optionally, the purified cell walls are neutralized, for example with dilute HCl, washed again with distilled water until neutrality, before drying dried.

The boiling processes are preferably repeated and/or conducted for some minimum time lengths, for example boiling in distilled water may be conducted for about 3 h and repeated three times. Boiling in NaOH is preferably conducted three times for about 2 h each time. The purified cell walls may then be rinsed with distilled water, neutralized with dilute HCl, washed again with distilled water until neutrality, and dried.

The cellulose obtained by the methods and devices of the invention is preferably the cellulose contained in said algae, preferably said coenocytic algae.

In an embodiment, said cellulose is microfibrillated cellulose (MFC) and/or nanocellulose.

In an embodiment, said cellulose exhibits one, several or all of the following characteristics:
- a degree of polymerisation of > 15'000, preferably ≥ 20'000;
- the presence of cellulose microfibrils having a length of 500-3000 nm, preferably 900-2500 nm, most preferably 1000-2000 nm;
- the presence of cellulose microfibrils having a thickness of 5-30 nm, preferably 7-25, preferably most preferably 10-20 nm.

While certain of the preferred embodiments of the present invention have been described and specifically exemplified above, it is not intended that the invention be limited to such embodiments. Various modifications may be made thereto without departing from the scope and spirit of the present invention, as set forth in the following claims. Herein below, examples of the invention are disclosed. These examples are for illustration only and are not intended to limit the scope of the present invention.

### Examples: Cultivation of coenocytic algae

A focus was put in this study on *Boergesnia forbesii* and *Valonia ventricosa* which are both *Cladophorales* with some fascinating characteristics such as (1) a thick and stratified cell wall, (2) multinucleate cells, (3) reproduction can be asexual (quadriflagellate zoospore or sexual (reproduction by isogamy), (4) isomorphic alternation of generation.

### 1. Materials and Methods

Coenocytic algae *Valonia ventricosa* (UTEX LB 2260) and *Boergesenia forbesi* (UTEX LB 2410) were purchased from UTEX (Culture Collection of Algae at the University of Texas at Austin) as a free cell in culture medium. Modified growth media was adapted from the ES/2 Seawater culture medium.

The chemical composition of the new media is provided below:
- For a total of 1 L of culture medium.
- Aseptically 5 mL of sterile modified ES2 Enrichment Solution was added per litre of sterilized Seawater (30 g/L).

**Table 2: Modified culture medium for coenocytic algae**

| **Component** | **Amount** |
|---|---|
| Sterilized seawater | 1 [L] |
| Enrichment solution for seawater medium | 5 [mL/L] |

**Table 3: Enrichment solution for seawater medium**

| **Component** | **Amount (/2[L])** |
|---|---|
| NaNO₃ (Fisher BP360-500) | 10 [g] |
| Na₂glycerophosphate•5H₂O (Sigma G 6501 ) | 0.5 [g] |
| ES FE Solution | 300 [mL] |
| P-II Metal Solution | 250 [mL] |
| HEPES buffer (Sigma H-3375) | 5 [g] |
| Vitamin B12 | 1 [mL] |
| Biotin Vitamin Solution | 1 [mL] |
| Thiamine Solution | 1 [mL] |

**Table 4: ES Fe solution recipe**

| **Component** | **Amount (/2[L])** |
|---|---|
| Fe(NH₄)₂(SO₄)₂•6H₂O (Sigma F-1513) | 1.4 [g] |
| Na₂EDTA•2H₂O (Sigma ED255) | 1.2 [g] |

**Table 5: P-II Metal solution recipe**

| **Component** | **Amount ( /100[mL])** |
|---|---|
| Na₂EDTA•2H₂O (Sigma ED255) | 0.1 [g] |
| H₃BO₃ (Baker 0084) | 0.114 [g] |
| FeCl₃•6H₂O (Sigma F-1513) | 4.9 [mg] |
| MnSO₄•H₂O (Sigma M8179) | 16.4 [mg] |
| ZnSO₄•7H₂O (Sigma Z 0251) | 2.2 [mg] |
| CoCl₂•6H₂O (Sigma C-3169) | 0.48 [mg] |

**Table 6: Vitamin B12 Solution component recipe**

| **Component** | **Amount (/200[mL] H₂0)** |
|---|---|
| HEPES buffer pH 7.8 (Sigma H-3375) | 2.4 [g] |
| Vitamin B₁₂ (cyanocobalamin, (Sigma V-6629)) | 0.015 [g] |

**Table 7: Biotin Vitamin Solution Component Recipe**

| **Component** | **Amount (/200[mL] H₂0)** |
|---|---|
| HEPES buffer pH 7.8 (Sigma H-3375) | 2.4 [g] |
| Biotin (Sigma B-4639) | 0.005 [g] |

**Table 8: Thiamine Vitamin Component Recipe**

| **Component** | **Amount (/200[mL] H₂0)** |
|---|---|
| HEPES buffer pH 7.8 (Sigma H-3375) | 2.4 [g] |
| Biotin (Sigma B-4639) | 0.30 [g] |

### 2. Growth condition effects in aplanospore production:

This step consists of finding the best conditions to produce aplanospore cells to prepare for the cell adhesion in a later step. Tested conditions were mainly temperature range (13°C, or 25 °C) and carbon source (urea 5mM, or acetate 5mM).

### a. Temperature:

Working under a laminar flow cabinet, 5 *Boergesenia forbesi* cells harvested from a preculture are transferred to a 15mL ES/2 modified medium. Warm-white LED plates with light intensity set at 55 [µmol photon/m²s] are fixed at the ground of two incubators, with two temperature ranges, respectively to 13°C and 25°C. The flasks are disposed in the incubators for 28 days; a slow circle-shaking device is turned on, imitating the sea behaviour. Every two days, pictures of the samples are taken.

At day 10, cells are pierced with needles, and the protoplasm is recovered in Eppendorfs for cytometry of aplanospore counting. Moreover, the remaining membranes are first washed with water milli-q before going through a drying step. This represents the MFC production yield.

### b. Carbon source:

Two different carbon sources were added to the culture medium (urea 5mM, or acetate 5mM). the aim of this experiment was to verify if the addition of typical carbon source used in algae culturing could improve the production yield of coenocytic algae. Control culture in this experiment was performed using 2% CO₂/air mix.

Warm-white LED plate with light intensity set at 55 µmol photon/m²s is fixed at the ground of the incubator, with a temperature set to 25°C (at lower temperature a complete inhibition of *Boergesenia forbesi* cells was observed). Working under a laminar flow cabinet, 5 *Boergesenia forbesi* cells from a preculture are transferred to modified culture medium containing the different carbon source and thus for a period of 28 days. A slow circle-shaking device is turned on, imitating the sea behaviour. At day 10, cells are pierced with needles, and the protoplasm is recovered in Eppendorfs for cytometry of aplanospore counting. Moreover, at the end of the experiment, the remaining membranes are first washed with water milli-q before going through a drying step. This represents the MFC production yield.

### 3. Results and discussion

### 3.1 Carbon source and Temperature effects

The impact of cold temperature is different according to the medium used. Cells in acetate enriched or control medium mostly survived but stopped their growth while all cells in enriched urea medium died, only aplanospores inside the membranes of the dead cells were found at the end of the experiment. Applying a mechanical stress at 13 °C did not trigger any aplanospore formation.

Cells that grew at 28°C and using acetate a carbon source created a numerous aplanospores cells after applying a mechanical stress. The results indicate that under these conditions, *Boergesnia forbesii* can produce aplanospores efficiently and without a net reduction in total biomass production in comparison to the urea medium. Less aplanospores were counted in the case of control compared to acetate, suggesting that the nature of carbon source could also play a role in aplanospore formation (238 spores/mL vs 375 spores/mL, respectively).

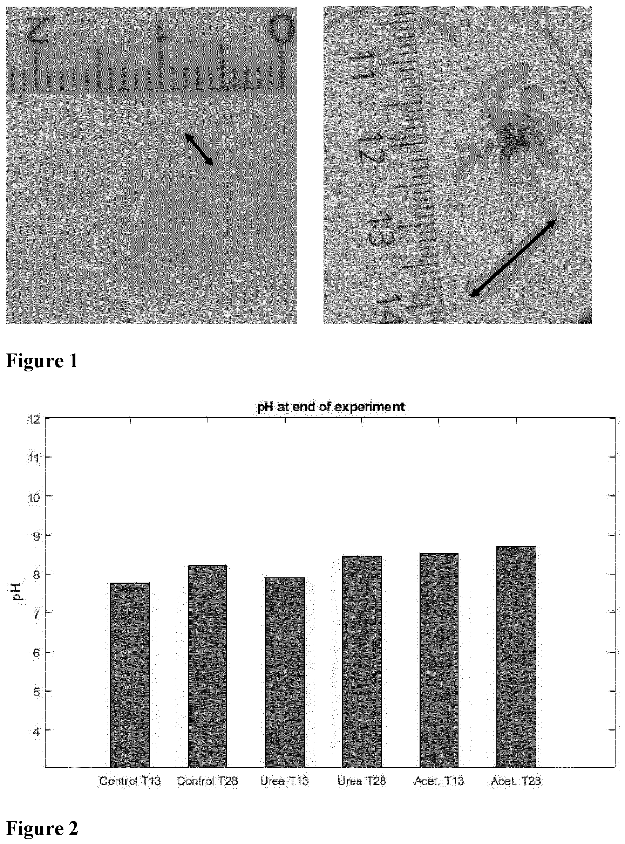

**Figure 1** shows two photographs, taken at day 1 and day 28 from a *B. forbesii* cell grown in a 5mM acetate medium, at 28°C. The double headed arrow shows a growth of the cell of 0.5 cm to 1.5 cm at day 28.

As shown in **Figure 2****,** pH increased with time, and this change is more noticeable at higher temperatures; the samples in the enriched acetate medium finished with the most elevated pH, followed by the enriched urea medium and the control medium with similar acidity.

**Table 9** below shows the growth rate determined for the 28-day period for the various treatments. Exponential growth was found in all cases, with highest growth rates found at higher temperature (28°C), and in the absence of urea.

**Table 9: Results of Growth rate/day in % of initial biomass**

| | Growth rate/day in % of initial biomass |
|---|---|
| **Medium:** Control, T: 28°C | 6.29 |
| **Medium:** Enriched with acetate, T: 28°C | 6.20 |
| **Medium:** Enriched with urea, T: 28°C | 2.84 |
| **Medium:** Control, T: 13°C | 2.4 |
| **Medium:** Control, enriched acetate, T: 13°C | 2.94 |
| **Medium:** Enriched with urea, T: 13°C | 1.37 |

### 3.2. Membrane analysis

The FTIR analysis (spectrum not shown) comparing the membranes recovered with wood MFC shows three main differences in the results. The first difference is a peak intensity around 1250 [cm⁻¹] higher for the samples when compared with the control cellulose; since the peak is present for every sample, independently of the experiment parameters, it is safe to assume that its presence is caused by the composition of the membrane. Some algae show a similar absorption band at this particular intensity, caused by the presence of P=O asymmetric stretching vibration in phosphodiesters. Another possibility is the presence of asymmetric stretching of S=O bounds that has been observed in many green algae's FTIR.

The second difference is also present for every sample and can be found at a peak intensity of 1640. Samples have a higher peak intensity than the control cellulose. The C=O stretching vibration of the amide-I protein that can be found in algae is the cause of this peak.

The last difference is less noticeable, it can be found around 3350cm⁻¹. Both samples and control cellulose have a peak at this wavelength, with the difference being in the higher absorbance intensity for the algae membrane. The peak is caused by the OH-O bonding next to the β-glycosidic bond of cellulose but can also be for algae related to the N-H stretching vibration, explaining the higher absorbance for algae membrane at this peak.

The FTIR shows that the membrane composition is very similar to pure cellulose, but small difference can be observed since some other components as protein are necessary to allow algae's exchange with its surrounding.

### 3.3. Solid Liquid (SL) support and aplanospore fixation

Tests were performed at early stage to check if aplanospore cells can attached again on natural stone and if this attachment improves the growth kinetics of coenocytic algae compared to a control culture where natural stone were not added.

**Figure 3** shows that within a period of 10 days, biomass increased significantly in the case of attached aplanospore compared to the control culture. Moreover, the cells diameters increased by a factor three (3) and chlorophyll content was significantly higher. These results suggest that the presence of CaCO₃ stone significantly improves the growth kinetics of coenocytic algae.

Three different supports were chosen in the experiments: natural stones (dead coral), Artificial SL made from CaCO₃ and finally SL-CaCO₃ dopped with iron. The best conditions of temperature and carbon source were selected from previous tests and used to perform SL support fixation test.

The results show the successful fixation of aplanospores cells on the natural stone and on the artificial SL made from CaCO₃. However, in the case SL-CaCO₃ dopped with iron showed a complete absence of attached aplanospore on the surface.

### 4. Artificial preparation of solid substrate for SL culture

Modified glass and limestone surfaces are used as a substrate, the surface should be rough enough to allow cell adhesion.

A solid glass surface is pre-treated by hydroxylation under UV light and the presence of ozone. In particular, a glass surface was treated under UV light using a solar simulator (Suntest, XLS+, UV A and B) for 1h. The treated glass was immersed in a MillQ solution bubbled with ozone for 2h to obtain hydroxylated surface.

The hydroxylated glass surface is immersed in a solution saturated with Ca²⁺ and CO₃²⁻ ions. The treated substrate is immersed in a bath saturated with Ca²⁺ and CO₃²⁻ ions under controlled conditions to trigger CaCO₃ deposition on the hydroxylated surface.

In general, the solution may be flowed to the substrate, agitated in any other way, or not agitated. The concentration of Ca²⁺ and CO₃²⁻ ions can be varied. The CaCO₃ deposition on the hydroxylated surface is a controlled deposition by heterogeneous nucleation and growth.

The controlled conditions produce any polymorphs of CaCO₃ including, but not limited to, calcite, aragonite, vaterite, and amorphous calcium carbonate. CaCO₃ seeds can be added to facilitate CaCO₃ nucleation and growth.

It is also possible to use CaCO₃ plate commercially available, but this reduces to possibility to control porosity and to add additional chemical to the surface.

The substrate can also be dopped with any other relevant nutrients for coenocytic algae growth, this includes but is not limited to Mg²⁺, Fe²⁺, and K⁺ ions (Only Fe²⁺ enriched CaCO₃ - SL was tested in this work).

### 5. Devices and methods for cultivating coenocytic green algae

Devices and methods for cultivating coenocytic green algae including the production of spores are conceived as shown in the figures.

## Claims

1. A method for cultivating coenocytic algae (4), the method comprising:
- providing a solid substrate (15) comprising coenocytic algae,
- and,
- cultivating said algae by exposing said substrate to a liquid culture medium (5).

2. The method of claim 1, wherein comprising obtaining said solid substrate (15) comprising coenocytic algae by inoculating a solid substrate with spores (6) of said coenocytic algae.

3. The method of claim 1, wherein said solid substrate comprises a carbonate mineral.

4. The method of claim 1 or claim 2, wherein said solid substrate comprises calcium carbonate (CaCO₃), preferably selected from the group consisting of: calcite, aragonite and vaterite, preferably at the surface of the substrate.

5. The method of any one of claims 2-4, wherein said solid substrate comprises said carbonate mineral deposited on a solid carrier material.

6. The method of claim 5, wherein said solid carrier material comprises negatively charged and/or hydroxylated substrates.

7. The method of any one of the preceding claims, wherein said solid substrate comprises glass coated with said carbonate mineral.

8. The method of any one of the preceding claims, wherein said solid substrate comprises a plate comprising two first and second opposed main surfaces (41, 42), on which said algae are inoculated, and wherein said substrate preferably comprises a polygonal, preferably quadrilateral outline.

9. The method of claim 8, wherein said first and second opposed surfaces of said solid substrate are preferably flat surfaces.

10. The method of any one of the preceding claims, wherein said liquid culture medium is supplemented with CO₂ and/or air during cultivation in said liquid culture medium.

11. The method of any one of the preceding claims, wherein said coenocytic algae are selected from chlorophyta, preferably from cladophorales.

12. The method of any one of the preceding claims, wherein said coenocytic algae are selected from *Chaetomorpha, Rhizoclonium, Microdyction, Valonia, Dictyosphaeria, Siphonocladus,* and *Boergesenia,* more preferably from *Valonia,* and *Boergesenia.*

13. The method of any one of the preceding claims, wherein cultivating said algae comprises exposing said algae to said liquid medium at a temperature of 23-35°C, more preferably 27-30°C.

14. The method of any one of the preceding claims, wherein said liquid culture medium comprises sea water.

15. A method for producing cellulose, the method comprising:
- cultivating coenocytic algae according to any one of claims 1-6;
- harvesting said cultivated coenocytic algae; and,
- extracting cellulose from said harvested algae.

16. The method of claim 15, wherein harvesting said cultivated coenocytic algae comprises mechanically removing biomass of said algae from said solid substrate.

17. The method of claim 15 or 16, wherein mechanically removing biomass comprises removing algae cells from said substrate while leaving roots of the algae in or on the substrate.

18. The method of any one of claims 15-17, which comprises:
- crushing said harvested algae;
- boiling the crushed algae;
- treating the crushed and boiled algae with alkaline solution; and,
- rinsing the treated algae, for example with water.

19. The method of any one of claims 15-18, wherein said cellulose is microfibrillated cellulose (MFC).

20. The method of any one of claims 15-19, wherein said cellulose exhibits one or more of the following characteristics:
- a degree of polymerisation of > 15'000, preferably ≥ 20'000;
- the presence of cellulose microfibrils having a length of 500-3000 nm, preferably 900-2500 nm, most preferably 1000-2000 nm;
- the presence of cellulose microfibrils having a thickness of 5-30 nm, preferably 7-25, preferably most preferably 10-20 nm.

21. A device (10) for cultivating coenocytic algae, the device comprising:
- a container (11) suitable to retain a liquid culture medium;
- one or more solid substrates (15) suitable to be placed in said container, wherein said solid substrate is suitable for growing said coenocytic algae thereon;
- channels, pipes, conducts and/or tubes (12) suitable for feeding gaseous CO₂ and/or air into the container and more preferably to said liquid culture medium in said container;
- a heater for heating said liquid culture medium, and,
- optionally: one or more light (14) sources for illuminating algae growing on said solid substrate.

22. The device of claim 21, wherein solid substrate is the substrate as defined in any one of claims 1-9.

23. A method for producing coenocytic algae containing spores and/or for producing spores of coenocytic algae, the method comprising:
- providing cells of said coenocytic algae and separating said cells in at least two groups on the basis of the size of said cells, a first group comprising comparatively larger cells (2) and a second group comprising comparatively smaller cells (1);
- cultivating said smaller cells (1) in a liquid medium (5) in first container;
- exposing said larger cells (2) to mechanical stress and continuing cultivating said larger cells in a second container (22);
- removing cells (3) comprising spores (6) from said second container and separating said spores (6) from said removed cells.

24. The method of claim 23, which comprises:
- growing cells of said coenocytic algae in said first container (21) before separating said cells in two groups.

25. The method of claim 23-24, wherein separating said cells comprises filtering said larger cells (2).

26. A device (20) for producing coenocytic algae containing spores and/or for producing spores of coenocytic algae, the device comprising:
- first and second containers (21, 22) suitable for cultivating said coenocytic algae in a liquid culture medium;
- a transfer device (25), suitable for transferring cells from said first to said second container;
- a separating device (23), suitable for separating larger cells from smaller cells; and,
- a cell removing device (24), suitable to remove spore-containing cells from said second container.

27. The device of claim 26, wherein said separating device (23) comprises a sieve, suitable for separating cells on the basis of their size.

28. The device of claim 26 or claim 27, wherein said cell removing device (24) comprises a wiper and/or scraper, provided to wipe and/or scrape cells from said second container, preferably from the sieve on claim 26.

29. The device of any one of claims 25-28, wherein said transfer device (25) comprises first and second cell transfer channels (26, 27),
wherein said first cell transfer channel (26) is provided to transfer cells from said first container to said second container, preferably by transferring liquid medium containing said cells, from said first to said second container and,
wherein said second cell transfer channels (27) is provided to transfer cells, preferably by transferring liquid medium containing said cells, from said second container to said first container.

30. The device of claim 29, wherein said first and second cell transfer devices comprise separate paths and/or channels for liquid transport.

31. The device of any one of claims 29-30, wherein one selected from said first and second cell transfer channels uses gravity as driving force for transferring cells between the containers.

32. The device of any one of claims 26-31, further comprising one or more light sources (31, 32) for illuminating cells in said first and second container and for supporting growth of said algae in said containers.

33. The device of any one of claims 26-32, further comprising a mechanical stress inducing entity (35) for exposing said cells to mechanical stress, wherein said stress inducing entity preferably comprising a plurality of spikes (36).
